# EUROPEAN PATENT SPECIFICATION

(11) **EP 3 011 222 B1**
(45) Date of publication and mention of the grant of the patent: **01.05.2019**
(21) Application number: 14752775.8
(22) Date of filing: 12.06.2014
(51) Int. Cl.: F16M 13/02, A61B 6/00

(54) **ARM OF CEILING HOLDER**
ARM EINER DECKENSTÜTZE
BRAS D'UN SUPPORT DE PLAFOND

(30) Priority: 18.06.2013 CZ 20130468
(43) Date of publication of application: 27.04.2016
(73) Proprietor: MZ Liberec, A.S., 46001 Liberec 3 (CZ)
(72) Inventor: Vanoucek, Jan, 512 36 Horní Branná (CZ)
(74) Representative: Skoda, Milan
(86) International application number: PCT/CZ2014/000065
(87) International publication number: WO 2014/202032

(56) References cited:
- FR-A1- 2 956 719
- US-A- 5 425 068
- US-A1- 2012 257 725

## Description

### Technical Field

The present invention relates to an arm of a ceiling holder, especially to the arm of the ceiling holder for attaching of pendant accessories such as medical devices, medical apparatuses, tools and equipment, which includes a supporting part and end pieces used for connection with the pendant accessories and/or other supporting parts of the ceiling holder.

### State of the Art

A wide range of the arm structures of ceiling holders is known from the art and they are used for attaching of the pendant accessories, such as medical devices, tools, and equipment in the operating theatres or examination rooms. The arms contain the supporting part made from a metal material.

The main disadvantage of the arms, which include metal supporting part, is increased weight that causes deteriorated manoeuvring with the arms. Higher weight goes hand in hand with higher rating of the handling and balancing structural components, which again increases total weight of the arm. Higher weight stands for more material and thereby, higher total price of the holder. Higher costs are also associated with necessity of higher rating of the holder fixing component in the ceiling structure.

Patent Document FR 2956719 describes a ceiling holder to bear devices and apparatuses used in operating theatres. The holder consists of a horizontal arm a part of which is designed as an elliptical cylinder made of resin and reinforced with carbon fibres. If the horizontal arm is designed and made of a composite tube only, its main disadvantage is its instability as it can easily be bent, especially if designed and made as an oval or circular tube. Another disadvantage is the fact that the cables inside the arm are not arranged in any way.

Another patent document (US 5425068) describes a supporting system for X-ray devices that includes an arch-shaped tilting arm suitable for bearing very heavy devices (such as X-ray ones). The whole arch-shaped tilting arm is sliding in guide pulleys in such a manner that both the opposite parts of the X-ray device remain permanently arranged one against the other in a stable position and they can rotate in 3D along with the arm as a whole. The disadvantage of this arm is the fact that it cannot be used for any purpose different from that described in this document.

The aim of the present invention is the structure of the arm of the ceiling holder, structure of which will be far lower when compared to the art, easier to manufacture and cheaper in general.

### Principle of the Invention

Said disadvantages are eliminate to a considerable extent and aims of the invention are accomplished by the arm of the ceiling holder, especially the arm of the ceiling holder for attaching of pendant accessories such as medical devices, medical apparatuses, tools and equipment, comprising of the supporting part connected with the pendant accessories and/or other supporting parts of the ceiling holder according to the present invention, wherein the substance of the invention lies in that the supporting part consists of a metal core, whereas the core is coated with the composite skeleton and the core comprises at least one inner divider inside the core.

The benefit of both embodiments is that the arm is very lightweight. At the same time, production of the arm is rather easy. In case the arm includes the core, the core is favourably wrapped in the material of the composite skeleton. This permits optimization of the arm properties with respect to its overall dimensions. The structure permits simplified production of the arms with a wide range of lengths, without extra demands for the production equipment. The composite skeleton favourably consists of carbon fibres in a binder. Most favourably and considering total weight, the core is made from an aluminium-based alloy.

Considering said optimization of the arm embodiment and considering to its properties and production costs, it is favourable for the composite skeleton to have a variable thickness. Most favourably, the composite skeleton is embodied so that its thickness is smallest roughly in the centre of the supporting part, more favourably the supporting part is thinnest in the centre between the arm parts behind which it is connected to another supporting parts of the holder and that arm part where ballast is fixed.

The inner divider is favourably an integral part of the composite skeleton. In another embodiment, the inner divider may be either additionally inserted or fixed, for example glued.

The supporting part has favourably circular shape. This shape is favourable with respect to its strength properties and production simplicity and is favourable with respect to maintenance of the ceiling holder with respect to its placement in the environment of operating theatres, which means in environments with increased sanitary demands. It may also be favourable for the supporting part to have squared shape, whereas the shape is four-sided or multiply sided.

Most favourably, the supporting part is connected with the pendant accessories and/or other supporting parts of the ceiling holder via at least one end piece. This embodiment permits very easy production and high variability of embodiments of arms of the ceiling holder. The end piece may for example comprise a joint for connection for other parts of the ceiling holder or pendant accessories.

It is favourable considering variability of the structure mentioned above if at least one part of the end piece is metal and/or composite. If the end piece or any part thereof is metal, the most favourable production method is casting.

The end piece is then favourably joined to the supporting part by pressing on and/or with a glue layer.

A great advantage of the arm of the ceiling holder according to the present invention is substantially lower weight compared to embodiments of arms in the art. This brings possibility of substantial lessening of handling and balancing components, thereby further reduction of total weight of the arm. This means substantial saving in material and thereby substantial reduction of total costs for production of the ceiling holder. Reduction of total weight also improves and simplifies manoeuvrability of the arms and allows reduction of requirements for loading capacity of attachment to the ceiling structure.

The structure of the arm of the ceiling holder according to the present invention is in addition highly variable, whereas it allows precise optimization of the arm properties with respect to its use. The structure also permits simplified production of the arms with a wide range of lengths, without extra demands for the production equipment.

### Overview of the Figures

The invention will be explained in detail based on the drawing, where Fig. 1 illustrates cross-section view embodiment of the supporting part with the core and the composite skeleton, Fig. 2 illustrates cross-section view embodiment of the supporting part with the core and composite skeleton, with the inner divider arranged in its cavity, Fig. 3 illustrates view to individual supporting parts of the arm of the ceiling holder, and Fig. 4 illustrates view on the arm of the ceiling holder ready for installation in the holder.

### Examples of the Performance of the Invention

### Example 1

The arm 1 of a ceiling holder for attaching of pendant accessories such as medical devices, medical apparatuses, tools and equipment, which includes the supporting part 2 connected with the pendant accessories and/or other supporting parts of the ceiling holder.

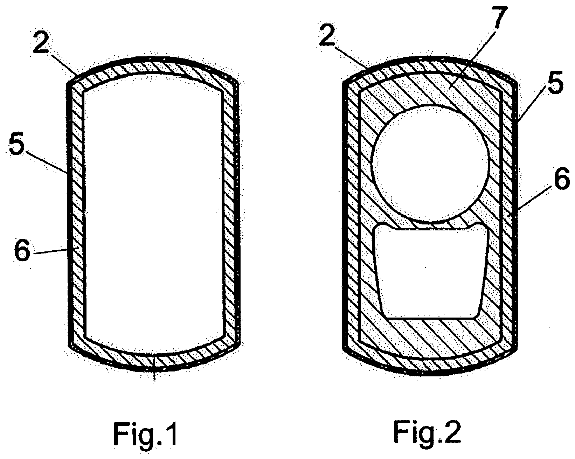

The supporting part 2 (Fig. 1) comprises of the metal core 6 coated with composite skeleton 5, whereas the supporting part 2 adapted on its ends for connection with other parts of the ceiling holder and attaching of the pendant accessories.

The composite skeleton 5 has variable thickness, whereas the thinnest section is roughly in the centre of the supporting part 2 between attaching points thereof.

In addition, the supporting part 2 (Fig. 2) comprises of the inner dividers 7 used for reinforcement of the supporting part and guiding of mechanisms inside the arm 1 cavity. The inner dividers 7 are attached by gluing inside the core 6 cavity.

The supporting part 2 has four-sided shape, whereas round shape may be used in another embodiment.

### Example 2

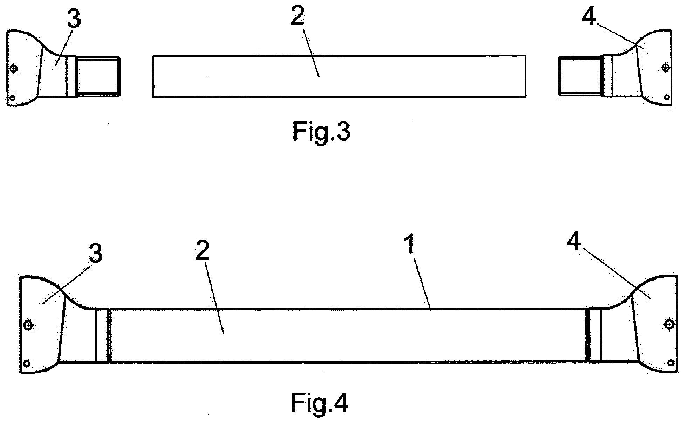

The arm 1 (Fig. 3, Fig. 4) of the ceiling holder for attaching of pendant accessories such as medical devices, medical apparatuses, tools and equipment includes the supporting part 2 connected with the pendant accessories and/or other supporting parts of the ceiling holder.

The supporting part 2 (Fig. 1) comprises of the metal core 6 coated with the composite skeleton 5, whereas the supporting 2 has the end pieces 3, 4 for connection with the pendant accessories and other supporting parts of the ceiling holder.

The composite skeleton 5 has variable thickness, whereas the thinnest section is roughly in the centre of the supporting part 2 between attaching points thereof.

In addition, the supporting part 2 (Fig. 2) comprises of the inner dividers 7 used for reinforcement of the supporting part and guiding of mechanisms inside the arm 1 cavity. The inner dividers 7 are attached by gluing inside the core 6 cavity.

The supporting part 2 has four-sided shape, whereas round shape may be used in another embodiment.

The end pieces 3, 4 are made from metal or composite material, whereas they are connected with the supporting part 2 by pressing.

### Industrial Application

The arm of the ceiling holder may particularly be used for attaching of pendant accessories in the operating theatres and examination rooms of health care facilities.

### List of Reference Marks

- 1: arm
- 2: supporting part
- 3: end piece I
- 4: end piece II
- 5: composite skeleton
- 6: core
- 7: inner divider

## Claims

1. An arm of a ceiling holder, especially the arm (1) of the ceiling holder for attaching of pendant accessories such as medical devices, medical apparatuses, tools and equipment, which includes a supporting part (2) connected with the pendant accessories and/or other supporting parts of the ceiling holder, **characterized by that** the supporting part (2) comprises of a metal core (6), whereas the core (6) is coated with the composite skeleton (5) and the core (6) comprises at least one inner divider (7) inside the core (6).

2. The arm of the ceiling holder according to claim 1 **characterized by that** the composite skeleton (5) has variable thickness.

3. The arm of the ceiling holder according to any one of preceding claims **characterized by that** the composite skeleton (5) has variable thickness, whereas the thinnest section is roughly in the centre of the supporting part (2).

4. The arm of the ceiling holder according to claim 1 **characterized by that** the inner divider (7) is an integral part of the composite skeleton (5).

5. The arm of the ceiling holder according to any one of preceding claims **characterized by that** the supporting part (2) has either circular or squared shape.

6. The arm of the ceiling holder according to any one of preceding claims **characterized by that** the supporting part (2) is connected with the pendant accessories and/or other supporting parts of the ceiling holder via at least one end piece (3, 4).

7. The arm of the ceiling holder according to claim 6 **characterized by that** at least a part of the end piece (3, 4) is made from a metal material.

8. The arm of the ceiling holder according to any of claims 6 and 7 **characterized by that** at least a part of the end piece (3, 4) is made from a composite material.

9. The arm of the ceiling holder according to any one of preceding claims **characterized by that** the end piece (3,4) is connected with the supporting part (2) by pressing and/or glue layer.

## Patentansprüche

1. Der Arm des Deckenstativs, insbesondere der Arm (1) des Deckenstativs für die Festhaltung des Aufhängzubehörs wie medizinische Geräte, ärztliche Instrumente, Hilfsmittel und Apparate, welches aus einem tragenden Teil (2) besteht, der mit dem Aufhängzubehör und/oder anderen tragenden Teilen des Deckenstativs verbunden ist, **ist dadurch gekennzeichnet, dass** der tragende Teil (2) einen Kern (6) enthält, wobei der Kern (6) mit einem zusammengesetzten Skelett (5) bezogen ist, und der Kern (6) mindestens eine Innentrennwand (7) innerhalb des Kerns (6) enthält.

2. Der Arm des Deckenstativs nach der Forderung 1, **ist dadurch gekennzeichnet, dass** das Skelett (5) eine variable Stärke aufweist.

3. Der Arm des Deckenstativs nach einer der vorherigen Forderungen, **ist dadurch gekennzeichnet, dass** das zusammengesetzte Skelett (5) eine variable Stärke hat, wobei diese ungefähr inmitten des tragenden Teils (2) am dünnsten ist.

4. Der Arm des Deckenstativs nach der Forderung 1, **ist dadurch gekennzeichnet, dass** die Innentrennwand (7) untrennbaren Bestandteil des zusammengesetzten Skeletts (5) bildet.

5. Der Arm des Deckenstativs nach einer der vorherigen Forderungen, **ist dadurch gekennzeichnet, dass** der tragende Teil (2) eine Kreis- oder quadratische Form hat.

6. Der Arm des Deckenstativs nach einer der vorherigen Forderungen, **ist dadurch gekennzeichnet, dass** der tragende Teil (2) mit dem Aufhängzubehör und/oder anderen tragenden Teilen des Deckenstativs mittels mindestens eines Endstücks (3,4) verbunden ist

7. Der Arm des Deckenstativs nach der Forderung 6, **ist dadurch gekennzeichnet, dass** mindestens ein Teil des Endstücks (3,4) metallisch ist.

8. Der Arm des Deckenstativs nach einer der vorherigen Forderungen, **ist dadurch gekennzeichnet, dass** mindestens ein Teil des Endstücks (3,5) zusammengesetzt ist.

9. Der Arm des Deckenstativs nach einer der vorherigen Forderungen, **ist dadurch gekennzeichnet, dass** das Endstück (3,4) mit dem tragenden Teil (2) durch Auspressen und/oder eine Klebstoffschicht verbunden ist.

## Revendications

1. Le bras du stative de plafond, notamment le bras (1) du stative de plafond pour la fixation des accessoires de suspension, comme les moyens de santé, les instruments médicaux, les outilles et les appareilles, qui contient la partie (2) portative liée à l'accessoire de suspension et/ou d'autres parties portatives du stative de plafond, **caractérisé par le fait, que** la partie (2) portative contient le noyau (6) métallique, et en même temps le noyau (6) est couvert par une couverture (5) composite et le noyau contient une cloison (7) intérieure à l'intérieur du noyau (6).

2. Le bras du stative de plafond selon la revendication 1, **caractérisé par le fait, que** la couverture (5) composite est de l'épaisseur variable.

3. Le bras du stative de plafond selon une des revendications précédentes, **caractérisé par le fait, que** la couverture (5) composite est de l'épaisseur variable et en même temps elle est la plus mince approximativement au milieu de la partie (2) portative.

4. Le bras du stative de plafond selon la revendication 1, **caractérisé par le fait, que** la cloison (7) intérieure est la partie intégrante de la couverture (5) composite.

5. Le bras du stative de plafond selon une des revendications précédentes, **caractérisé par le fait, que** la partie (2) portative a la forme circulaire ou anguleux.

6. Le bras du stative de plafond selon une des revendications précédentes, **caractérisé par le fait, que** la partie (2) portative est lié à l'accessoire de suspension et/ou d'autres parties portatives du stative de plafond par l'intermédiaire d'une fermeture (3,4) au moins.

7. Le bras du stative de plafond selon la revendication 1, **caractérisé par le fait, qu'**au moins une partie de la fermeture (3,4) est métallique.

8. Le bras du stative de plafond selon une des revendications 6 et 7, **caractérisé par le fait, qu'**au moins une partie de la fermeture (3,4) est composite.

9. Le bras du stative de plafond selon une des revendications précédentes, **caractérisé par le fait, que** la fermeture (3,4) est liée à la partie (2) portative par un montage fait à la presse et/ou par une couche de colle
